# EUROPÄISCHE PATENTSCHRIFT

(11) **EP 4 144 300 B1**
(45) Veröffentlichungstag und Bekanntmachung des Hinweises auf die Patenterteilung: **27.11.2024**
(21) Anmeldenummer: 22192009.3
(22) Anmeldetag: 24.08.2022
(51) Int. Cl.: A61B 8/00

(54) **AUSWECHSELBARER AUFSATZ FÜR EINE ULTRASCHALLSONDE**
REPLACEABLE HEAD FOR AN ULTRASONIC PROBE
ADAPTATEUR INTERCHANGEABLE POUR UNE SONDE À ULTRASONS

(30) Priorität: 02.09.2021 CH 0702312021
(43) Veröffentlichungstag der Anmeldung: 08.03.2023
(73) Patentinhaber: Compremium AG, 3074 Muri b. Bern (CH)
(72) Erfinder: BAUMANN, Ulrich André, 3110 Münsingen (CH); BAUMANN, Vincent Boris, 3073 Gümligen (CH); FREI, Peter Nuot, 4657 Dulliken (CH)
(74) Vertreter: Keller Schneider Patent- und Markenanwälte AG

(56) Entgegenhaltungen:
- WO-A1-2016/126257
- US-A- 6 132 378
- US-A1- 2010 234 733
- US-A1- 2011 087 105
- US-A1- 2014 163 382
- US-A1- 2020 383 660

## Beschreibung

### Technisches Gebiet

Die Erfindung betrifft einen auswechselbaren Aufsatz für eine Ultraschallsonde mit einem ringartigen Befestigungsbereich zur Befestigung des Aufsatzes an der Ultraschallsonde und einer am Befestigungsbereich angeordneten flexiblen Membran. Ein erstes flüssiges oder gelartiges Kontaktmedium ist auf einer innenseitigen I<ontal<tfläche der Membran aufgebracht, wobei die I<ontal<tfläche bei an der Ultraschallsonde befestigtem Aufsatz eine Stirnseite der Ultraschallsonde kontaktiert. Die Erfindung betrifft weiter einen verpackten Aufsatz für eine Ultraschallsonde und eine Ultraschallsonde mit einem Aufsatz.

### Stand der Technik

Die Sonografie ist als bildgebendes Verfahren zur Untersuchung von organischem Gewebe in der Medizin und Veterinärmedizin gut etabliert. Gängigerweise kommt eine Ultraschallsonde zum Einsatz, die über die I<örperoberfläche geführt wird. Die Sonde umfasst einen Ultraschallkopf zur Erzeugung von Ultraschallwellen, beispielsweise mittels eines Piezoarrays. Diese werden über eine Stirnfläche des Ultraschalll<opfs in den menschlichen oder tierischen Körper eingekoppelt. Die reflektierten Signale werden wiederum von der Ultraschallsonde erfasst, und gestützt auf die Laufzeit der reflektierten Signale können die Tiefen der reflektierenden Gewebestrukturen bestimmt werden. Daraus lassen sich dann die gewünschten Bilddaten aufbereiten, wobei anhand der Echosignale die Helligkeit der Bildinformation bestimmt wird.

Zur Sicherstellung der Einkopplung der Ultraschallwellen in den Körper wird üblicherweise ein Kontaktmedium, z. B. ein Hydrogel, eingesetzt, das auf die Stirnfläche der Ultraschallsonde (oder die Haut) aufgetragen wird.

Dieselbe Ultraschallsonde wird nacheinander zur Untersuchung mehrerer Patienten eingesetzt. Es muss somit sichergestellt werden, dass über die Ultraschallsonde keine Keime von einem Patienten auf einen anderen übertragen werden. Gängigerweise erfolgt dies durch die Desinfektion der Ultraschallsonde zwischen den Untersuchungen, wobei in der Regel geeignete Desinfektionsmittel manuell, mittels eines Schwamms oder eines Tuchs, angewandt werden; in gewissen Fällen ist auch ein Eintauchen in das Desinfektionsmittel möglich. Der Grad der Desinfektion wird in der Regel auf die geplante Verwendung, also z. B. die Interaktion mit intakter Haut, die Interaktion mit Schleimhäuten oder den intraoperativen oder laparoskopischen Einsatz, abgestimmt. Zudem werden sterile Kontaktmedien eingesetzt. Eine Sterilisation durch Hitze ist aufgrund der Empfindlichkeit der Komponenten der Ultraschallsonde nicht möglich.

Der Prozess der Desinfektion ist relativ zeitaufwendig und kann bei nicht sachgemässer oder nicht an die Untersuchung angepasster Durchführung das Risiko der Übertragung von Keimen nicht beseitigen. Es ist aus dem Stand der Technik deshalb bekannt, den Ultraschallkopf bzw. dessen Stirnseite mit einem auswechselbaren sterilen Aufsatz zu versehen, der zwischen aufeinanderfolgenden Untersuchungen verschiedener Patienten ausgewechselt wird (single-use bzw. single-patient use).

So zeigt die US 2010/0234733 A1 (P. Wahl heim) beispielsweise einen sterilen schlauchartigen Überzug, in dessen geschlossenem Endbereich ein Abteil mit einem Kontaktmedium vorgesehen ist. Wird der Überzug auf die Ultraschallsonde aufgezogen, reisst das Abteil auf und gibt das Kontaktmedium frei.

Einen ähnlichen Überzug zeigt die US 5,997,481 (Ultra Sound Probe Covers, LLC). Bei diesem wird das Abteil durch eine flexible Membran gebildet, die eine Öffnung aufweist, damit das I<ontal<tgel in den Zwischenraum zwischen der Membran und der Stirnseite des Ultraschalll<opfs fliessen kann.

Die Handhabung der Überzüge, die die gesamte Ultraschallsonde und zum Teil sogar das daran angeschlossene Kabel bedecken, ist allerdings aufwendig. Zudem ist jeweils eine relativ grosse Menge an Kontaktmedium notwendig, die sich zudem weitgehend frei im Bereich des Ultraschallkopfs verteilen kann. Eine gleichbleibend gute Signalqualität kann so nicht sichergestellt werden.

Die US 6,132,378 A (Sharon Marino) beschreibt eine Haube für eine Ultraschallsonde, wobei die Haube über die Ultraschallsonde gestülpt werden kann. Eine flexible, mit einer Flüssigkeit oder einem Gel gefüllte Membran ist innerhalb der Haube angebracht.

Die US 2014/0163382 (Sound Technology Inc.) wiederum offenbart eine Abdeckung für einen Ultraschallwandler, welche eine Basis und eine schalldurchlässige Membran mit einem schalldurchlässigen Klebstoff umfasst, der wiederum mechanisch und akustisch mit der Membran in Verbindung steht.

Die WO 2016/126257 (Sound Technology Inc.) betrifft ebenfalls eine Abdeckung für ein Ultraschallgerät mit einer schalldurchlässigen Membran, einer Halterung für die Membran und einem Klebstoff, welcher auf der Membran angeordnet ist.

Die US 2011/0087105 A1 (Soma Development, LLC) beschreibt ein Gehäuse für einen Ultraschallwandler, welches den gemeinsamen Einsatz einer medizinischen Sonde und eines Ultraschallwandlers unterstützt/erlaubt.

Die WO 2019/147940 A1, auch veröffentlicht als US 2020/0383660 A1, (Butterfly Network, Inc.) beschreibt einen Aufsatz für eine Ultraschallsonde, der ein Behältnis für ein Kontaktgel umfasst, das über Öffnungen zwischen dem Behältnis und der dem Ultraschallkopf zugewandten Seite und/oder der Aussenseite verfügt. Das Kontaktgel wird somit auf der I<ontaktfläche zwischen dem Aufsatz und der Stirnseite des Ultraschallkopfs und/oder auf der I<ontaktfläche zwischen dem Aufsatz und der I<örperoberfläche verteilt und unterstützt so die Übertragung der Ultraschallwellen.

Allerdings ergeben sich durch das Behältnis zwei zusätzliche Grenzflächen, die grundsätzlich das Ultraschallsignal und die reflektierten Signale beeinträchtigen. Auch die Lochstruktur kann in dieser Hinsicht nachteilig sein. Das Behältnis muss zudem relativ viel Gel enthalten, damit eine ausreichende Benetzung der I<ontaktflächen sichergestellt ist, was wiederum die Signalqualität beeinträchtigt.

### Darstellung der Erfindung

Aufgabe der Erfindung ist es, einen dem eingangs genannten technischen Gebiet zugehörenden Aufsatz zu schaffen, der einfach zu handhaben ist und eine hohe Signalqualität ermöglicht.

Die Lösung der Aufgabe ist durch die Merkmale des Anspruchs 1 definiert. Gemäss der Erfindung ist vor dem Befestigen des Aufsatzes das erste Kontaktmedium auf der I<ontaktfläche derart verteilt, dass in einem Arraybereich der I<ontaktfläche eine Menge des ersten Kontaktmediums pro Fläche mindestens doppelt so gross ist wie in einem übrigen Bereich der I<ontaktfläche. Dabei ist eine Positionierung des Arraybereichs einer Positionierung eines Piezoarrays der Ultraschallsonde angepasst und eine Fläche des Arraybereichs beträgt mindestens 60% und maximal 175% einer Fläche des Piezoarrays.

Der Aufsatz ist insbesondere zur einmaligen Nutzung (single-use) bzw. zur exklusiven Nutzung für einen Patienten, während einer Behandlungssequenz (single-patient-use) bestimmt, d. h. er wird nach Vornahme der Messung bzw. nach Abschluss der Behandlungssequenz oder vor dem Wechsel zu einem anderen Patienten von der Ultraschallsonde entfernt.

Der Befestigungsbereich dient insbesondere zur umlaufenden Befestigung an der Ultraschallsonde, wobei die zusammenwirkenden Befestigungsmittel des Aufsatzes und der Sonde über den gesamten Umfang oder bereichsweise miteinander zusammenwirken können. Der Befestigungsbereich kann an einem ringartigen Rahmen mit einer gewissen Formstabilität ausgebildet sein, oder er wird beispielsweise durch einen mit der Membran einstückig ausgebildeten Abschnitt mit grösserer Wandstärke gebildet. Letzterer kann eine hohe Flexibilität aufweisen. Die Geometrie des Rahmens oder des Abschnitts kann bespielsweise kreisringförmig sein, elliptisch, regelmässig oder unregelmässig polygonal, gegebenenfalls mit abgerundeten Eckbereichen.

Die Befestigungsmittel sind so ausgebildet, dass sie einen abgeschlossenen Raum zwischen der Membran des Aufsatzes und der Ultraschallsonde schaffen. Verschiedenartige Befestigungsmittel sind im Rahmen der Erfindung einsetzbar, so z. B. Clipgeometrien, zusammenwirkende Gewinde, Geometrien zur Schaffung einer BajonettVerbindung, magnetische Elemente, lösbare Klebeverbindungen oder ein Presssitz, der z. B. hergestellt wird, wenn der ringförmige Befestigungsbereich aus einem elastischen Material gebildet ist und dieser beim Aufziehen auf einen Gegenabschnitt der Ultraschallsonde gedehnt wird.

Die flexible Membran ist insbesondere umlaufend am Befestigungsbereich befestigt. Sie ist insbesondere vor dem Aufsetzen des Aufsatzes ungedehnt sowie wellen- und faltenfrei am Befestigungsbereich angeordnet. Der freie Bereich der Membran, d. h. derjenige Bereich, der bei am Befestigungsbereich angeordneter Membran auf beiden Hauptflächen zugänglich ist, ist bevorzugt kreisförmig. So ergibt sich bei einem isotropen Membranmaterial eine gleichmässige, isotrope Verteilung der auf die Membran einwirkenden Kräfte.

Die Membran ist so ausgebildet, dass sie sich beim Anbringen des Aufsatzes auf die Ultraschallsonde bestmöglich an die Stirnseite der Ultaschallsonde anschmiegt. Bevorzugt weist die Membran eine Dicke von 0.01-0.5 mm, bevorzugt 0.025-0.2 mm, besonders bevorzugt 0.035-0.065 mm, auf. Dünnere Membranen sind insofern bevorzugt als sie die Abbildungsqualität minimal beeinflussen und eine besonders grosse Flexibilität ermöglichen, was insbesondere dann von Vorteil ist, wenn die Ultraschallsonde einen Drucksensor aufweist, um (über die Membran) einwirkende Druckkräfte zu messen. Geeignete Materialien für die Membran sind beispielsweise Silikon oder Nitrilgummi (NBR). Die Shore-Härte des Materials beträgt 50 Shore A oder weniger, insbesondere 30 Shore A oder weniger.

Als "Kontaktfläche" wird hier einzig derjenige Bereich der Membran verstanden, der bei befestigtem Aufsatz in Kontakt mit der Stirnfläche der Ultraschallsonde steht. Sie kann vor dem Aufsetzen eben sein, und nach dem Aufsetzen der Geometrie der Stirnfläche der Ultraschallsonde entsprechend verformt. Die Stirnfläche bezeichnet dabei jene Fläche der Ultraschallsonde, die bei der Untersuchung eines Patienten über die Membran mit dessen I<örperoberfläche zusammenwirkt und über die die Ultraschallwellen und die reflektierten Signale aus- bzw. eingekoppelt werden. Die I<ontal<tfläche entspricht insbesondere im Wesentlichen dem gesamten freien Bereich der Membran, d. h. die Membran wirkt ausschliesslich mit der Stirnfläche der Ultraschallsonde zusammen.

Das erste Kontaktmedium stellt sicher, dass eine zuverlässige Übertragung von Ultraschallinformationen zwischen der Membran und der Ultraschallsonde stattfindet. Idealerweise ist das erste Kontaktmedium gelartig mit einer Viskosität, die verhindert, dass die im Arraybereich aufgebrachte Menge vorzeitig zerfliesst. Die Substanz für das erste Kontaktmedium wird zudem mit Vorteil so gewählt, dass die Membran auf der Stirnseite der Ultraschallsonde gleiten kann und die Bildung von Falten vermieden wird.

Insbesondere ist das erste Kontaktmedium im Arraybereich gleichmässig verteilt, d. h. eine erste Dicke der durch das erste Kontaktmedium gebildeten Schicht ist dort im Wesentlichen konstant. In einem übrigen Bereich der I<ontal<tfläche ist eine zweite Dicke des ersten Kontaktmediums entsprechend höchstens halb so gross wie die erste Dicke. Bevorzugt ist das Mengenverhältnis zwischen dem Arraybereich und dem übrigen Bereich vor dem Anbringen des Aufsatzes an der Ultraschallsonde mindestens 4:1, besonders bevorzugt mindestens 8:1.

Dass der Arraybereich an den Piezoarray der Ultraschallsonde angepasst ist, kann bedeuten, dass eine Fläche, Form und Position des Arraybereichs mit der Fläche, Form und Positionierung des Piezoarrays übereinstimmen. Die Fläche des Arraybereichs kann aber auch etwas grösser sein als diejenige des Piezoarrays, um sicherzustellen, dass der ganze Arraybereich im zusammengesetzten Zustand vom ersten Kontaktmedium bedeckt ist. Die Fläche des Arraybereichs kann auch etwas kleiner sein als diejenige des Piezoarrays, wenn davon ausgegangen wird, dass das erste Kontaktmedium beim Zusammensetzen aufgrund des darauf ausgeübten Drucks etwas nach aussen getrieben wird. Erfindungsgemäss beträgt eine Fläche des Arraybereichs mindestens 60% der Fläche des Piezoarrays und maximal 175% der Fläche des Piezoarrays. In beiden Fällen ist es von Vorteil, wenn der Arraybereich und die Fläche des Piezoarrays gegenseitig zentriert sind.

Der Aufsatz ermöglicht eine einfache und hygienische Handhabung der Ultraschallsonde. Durch die präzise Positionierung des ersten Kontaktmediums auf dem Aufsatz kann die Menge an Kontaktmedium zwischen der Membran und dem Ultraschallkopf reduziert werden. Es ergibt sich im Arraybereich ein vollflächiger Kontakt und die Bildgebung störende Luftblasen werden vermieden. Insgesamt resultieren bessere, stabilere und konstantere Bildinformationen. Die erfindungsgemässe Lösung ermöglicht zudem eine einfache und sichere Benutzung, insbesondere weil sich ein manueller Auftrag von Kontaktmedium zwischen Aufsatz und Ultraschallsonde erübrigt.

Mit Vorteil ist der Arraybereich im Wesentlichen ganzflächig vom ersten Kontaktmedium bedeckt, und der übrige Bereich der I<ontaktfläche ist im Wesentlichen frei von Kontaktmedium.

Bevorzugt bedeutet die Anpassung der Positionierung und Ausdehnung des Arraybereichs an die Positionierung und Ausdehnung des Piezoarrays, dass der Arraybereich im Wesentlichen rechteckig ist und das Zentrum des Rechtecks (also der Schnittpunkt seiner Diagonalen) mit dem Zentrum der I<ontaktfläche zusammenfällt. Viele gängige Ultraschallsonden umfassen ein Piezoarray mit einer im Wesentlichen rechteckigen Geometrie. Ein Verhältnis zwischen der Breite und Länge des Arrays beträgt dabei in der Regel mindestens 1:2. Entsprechend ist es von Vorteil wenn der Arraybereich dieser Geometrie entspricht. "Im Wesentlichen rechteckig" schliesst auch eine rechteckige Form mit abgerundeten Ecken ein oder eine längliche Geometrie, bei der die Längs- und/oder Querseiten geringfügig konkav und/oder konvex ausgestaltet sind. Mit Vorteil entspricht das Verhältnis zwischen der Breite und Länge des Arraybereichs dem Verhältnis zwischen der Breite und Länge des Piezoarrays im Rahmen einer maximalen Abweichung von ±25%.

Mit Vorteil ist auf einer Aussenfläche der Membran vor dem Befestigen des Aufsatzes ein zweites Kontaktmedium aufgebracht. Das zweite Kontaktmedium stellt sicher, dass eine zuverlässige Übertragung von Ultraschallinformationen zwischen der Membran und der I<örperoberfläche des Patienten stattfindet.

Grundsätzlich kann es sich beim zweiten Kontaktmedium um dieselbe Zusammensetzung handeln wie beim ersten Kontaktmedium. Die Medien können sich aber auch voneinander unterscheiden. Ein geeignetes Kontaktmedium ist beispielsweise ein Gel auf Wasserbasis (Hydrogel) mit einem Feuchthaltemittel, einem Verdickungsmittel sowie gängigen Konservierungs- und Neutralisationsmitteln. Im Handel sind geeignete Substanzen erhältlich, z. B. das Produkt SONOGEL Steril Ultraschall-Gel von der Sonogel Vertriebs GmbH, Bad Camberg, Deutschland.

Der Auftrag des zweiten Kontaktmediums vereinfacht die Handhabung der medizinischen Untersuchung weiter. Nach dem Anbringen des Aufsatzes ist sie unmittelbar betriebsbereit. Es ist zudem sichergestellt, dass die Menge und die Zusammensetzung des zweiten Kontaktmediums optimal an die Membran angepasst sind.

Mit Vorteil umfasst der Aufsatz ein Garantieelement mit einer Geometrie, die dazu angepasst ist, bei einem Abziehen des an der Ultraschallsonde befestigten Aufsatzes mit einer Aufsetzgeometrie der Ultraschallsonde so zusammenzuwirken, dass das Garantieelement mechanisch beschädigt wird. Entsprechend lässt sich am Aufsatz erkennen, ob dieser bereits einmal verwendet wurde - unerwünschte mehrmalige Verwendungen können somit ausgeschlossen werden.

Beim Garantieelement handelt es sich insbesondere um einen Garantiering. Dessen Geometrie weist insbesondere Sollbruchstellen auf, die beim Abziehen aufgerissen werden. Der Garantiering kann ganz abgerissen werden und gegebenenfalls an der Ultraschallsonde verbleiben. Bevorzugt wird er aber nur teilweise abgerissen, so dass die erfolgte Verwendung unmittelbar sichtbar ist, der Garantiering aber am Aufsatz verbleibt. Der Garantiering kann gleichzeitig den Befestigungsbereich bilden, er kann aber auch von diesem unabhängig sein.

Beim Garantieelement kann es sich auch um die flexible Membran des Aufsatzes handeln, wenn diese so ausgebildet ist, dass sie beim Abziehen des Aufsatzes beschädigt wird. Dazu kann sie mit entsprechenden Elementen der Ultraschallsonde zusammenwirken.

Beim Garantieelement kann es sich aber auch um einen Datenspeicher handeln, der beim Abziehen zerstört bzw. funktionsuntüchtig gemacht wird, so dass er sich anschliessend nicht mehr auslesen lässt. In einer weiteren Variante verhindert das Garantieelement nach erfolgtem Abziehen den Aufbau eines gewissen Vordrucks zwischen der Membran und dem Ultraschallkopf, wenn der Aufsatz erneut aufgesetzt wird.

Die Erfindung betrifft auch einen verpackten Aufsatz, umfassend einen erfindungsgemässen Aufsatz und eine Verpackung. Die Verpackung weist einen becherartigen Grundteil und einen Deckel auf. Dabei ist eine Innengeometrie des becherartigen Grundteils einer Aussengeometrie des Aufsatzes angepasst, so dass dieser passend im Grundteil aufnehmbar ist. Der Deckel weist eine Aussengeometrie auf, die einer Innengeometrie des Aufsatzes angepasst ist, so dass der Deckel bei im Grundteil aufgenommenem Aufsatz passend in den Aufsatz einsetzbar ist.

Der Aufsatz wird somit in einer klar definierten Position in der Verpackung gehalten. Die Verpackung schützt den Aufsatz vor einer Kontamination und vor dem Austrocknen des ersten und gegebenenfalls zweiten Kontaktmediums.

Mit Vorteil ist die Verpackung so ausgestaltet, dass der darin aufgenommene Aufsatz am Ultraschallkopf angeordnet werden kann, indem letzterer von oben in die (geöffnete) Verpackung eingeführt wird, dort den Aufsatz erfasst und mit dessen Befestigungsmitteln derart zusammenwirkt, dass der Aufsatz beim Zurückziehen des Ultraschalll<opfs aus der Verpackung an diesem verbleibt, insbesondere unmittelbar in der endgültigen Aufsetzposition.

Mit Vorteil weist der Deckel oder der Grundteil auf einer Innenseite, die bei in der Verpackung aufgenommenem Aufsatz parallel zur Membran des Aufsatzes verläuft, eine Ausnehmung auf, deren Geometrie einer Geometrie des Arraybereichs angepasst ist.

Die Ausnehmung schafft somit Platz für die Aufnahme des ersten Kontaktmediums. Bevorzugt ist die Ausnehmung in der Innenseite des Deckels ausgebildet. In diesem Fall kontaktiert der Deckel die innere Seite der Membran und damit deren I<ontal<tfläche.

Bevorzugt wird bei zwischen dem Grundteil und dem Deckel angeordneten Aufsatz die I<ontal<tfläche des Aufsatzes ausserhalb eines durch die Ausnehmung definierten Bereichs derart vollflächig kontaktiert, dass ein Auslaufen des ersten Kontaktmediums aus dem Arraybereich in den übrigen Bereich gehemmt wird.

Der Bereich mit einer vollflächigen Kontaktierung umschliesst die Ausnehmung vollständig, so dass ein Auslaufen des Kontaktmediums aus der durch die Ausnehmung definierten Region gehemmt wird. Der Bereich kann die gesamte I<ontal<tfläche ausserhalb des Arraybereichs vollflächig kontaktieren, oder es ist lediglich ein Wandbereich aussen angrenzend an den Arraybereich vorhanden, wo die vollflächige Kontaktierung stattfindet. Bevorzugt führt die beschriebene Geometrie dazu, dass das Auslaufen des ersten Kontaktmediums aus dem Arraybereich bei verpacktem Aufsatz während der maximalen Lagerungszeit im Wesentlichen verhindert wird.

Mit Vorteil ist im Grundteil eine Menge des zweiten Kontaktmediums enthalten, so dass die Aussenfläche der Membran des aufgenommenen Aufsatzes grossflächig im zweiten Kontaktmedium eingetaucht ist.

Dies stellt sicher, dass die Ultraschallsonde mit montiertem Aufsatz unmittelbar einsatzbereit ist, wobei die Aussenfläche der Membran bereits mit dem benötigten Kontaktmedium, z. B. I<ontal<tgel, versehen ist. Die Aussenfläche ist insbesondere ganzflächig im zweiten Kontaktmedium eingetaucht, so dass die Sonde unabhängig von der Orientierung der Verpackung auf ihrer gesamten, von der Membran gebildeten, Stirnfläche ausreichend mit dem zweiten Kontaktmedium benetzt ist.

Die Menge des zweiten Kontaktmediums im Grundteil kann so bemessen sein, dass ein einfaches Nachbenetzen des Aufsatzes möglich ist. Dazu wird der (an der Ultraschallsonde angebrachte) Aufsatz nochmals in den Grundteil eingeführt und somit mit der Aussenfläche der Membran ganz oder teilweise nochmals in das zweite Kontaktmedium eingetaucht.

Mit Vorteil weist der Befestigungsbereich des Aufsatzes eine Kontur zum Zusammenwirken mit einer Gegenkontur der Ultraschallsonde auf, wobei die Kontur und die Gegenkontur so ausgebildet sind, dass der Aufsatz nur in einer oder zwei einander entgegengesetzten Orientierungen auf die Ultraschallsonde aufsetzbar ist.

Bei der Kontur und der Gegenkontur kann es sich um Umfangskonturen, Vorsprünge, Ausnehmungen usw. handeln. Die Kontur kann auch mit einer Gegenkontur der Verpackung zusammenwirken, z. B. um sicherzustellen, dass die Ausnehmung im Deckel bzw. im Grundteil mit ihrer Orientierung auf den Arraybereich ausgerichtet ist. Falls der Arraybereich achsensymmetrisch zu einem Durchmesser der Membran ist, ergibt sich sowohl bei einer ersten Orientierung, als auch bei einer gegenüber diesen um 180° gedrehten Orientierung eine korrekte Ausrichtung. Bei einem nicht entsprechend achsensymmetrischen Arraybereich sollte die Orientierung eindeutig festgelegt sein.

Bevorzugt sind die Kontur und Gegenkontur so ausgebildet, dass beim Aufsetzen der Aufsatz so an der Ultraschallsonde einrastet, dass dieses Einrasten hörbar und/oder haptisch wahrnehmbar ist. Der Benutzer weiss dann unmittelbar, dass der Aufsatz vollständig auf die Ultraschallsonde aufgesetzt wurde.

Mit Vorteil weist die Stirnseite der Ultraschallsonde eine konvexe Geometrie auf, und die Membran des Aufsatzes wird beim Aufsetzen gemäss der konvexen Geometrie verformt.

Die Membran wird insbesondere elastisch verformt. Die Verformung führt insbesondere dazu, dass sich die Membran grossflächig an die Stirnseite der Ultraschallsonde anschmiegt. In Kombination mit dem ersten Kontaktmedium und dessen Verteilung wird dadurch erreicht, dass Ultraschallsignale zuverlässig zwischen Membran und Ultraschallsonde übertragen werden können.

Der erfindungsgemässe Aufsatz eignet sich insbesondere für den Einsatz mit einem Ultraschallkopf mit integriertem Drucksensor. Derartige Vorrichtungen sind bekannt, vgl. z. B. CH 707 046 A2 (VeinPress GmbH), WO 2019/106535 A1 (U. A. Baumann, V. Baumann). Entsprechende Druck- bzw. Kraftinformationen sind beispielsweise für die Ermittlung des Venendrucl<s oder elastischer Eigenschaften von untersuchtem Körpergewebe nützlich. Dazu muss der Druck beispielsweise in einem Messbereich von 5-500, insbesondere 5-250 mmHg präzise bestimmt werden können. Die Minimierung der Menge des ersten Kontaktmediums zwischen Membran und Ultraschallkopf trägt auch zu präzisen Druckmessungen bei.

Wenn der Ultraschallkopf einen Drucksensor aufweist, wird mit Vorteil durch die Verformung der Membran eine Vorspannung in der Membran aufgebaut, wobei mit dem Drucksensor ein über den aufgesetzten Aufsatz aufgrund der Vorspannung auf die Ultraschallsonde ausgeübter Ausgangsdruck gemessen werden kann.

Der Ausgangsdruck wird gemessen, ohne dass eine zusätzliche äussere Kraft auf die Membran einwirkt. Der Drucksensor weist entsprechend einen Messbereich und eine Messgenauigkeit auf, die eine präzise Messung dieses Ausgangsdrucks ermöglichen. Der Drucksensor kann bei entsprechendem Messbereich zusätzlich zur Messung der Vorspannung auch generell zum Messen eines Drucks zwischen der Ultraschallsonde und der I<örperoberfläche, während der Benutzung der Ultraschallsonde, genutzt werden.

Durch die vorgegebene Geometrie des Aufsatzes und der Ultraschallsonde sowie die bekannte Menge an Kontaktmedium zwischen der Membran und der Stirnfläche der Sonde ergibt sich bei jedem Aufsetzen im Wesentlichen dieselbe Situation, d. h. ein Soll-Ausgangsdruck ist bekannt.

Ganz generell ist, unabhängig von der erfindungsgemässen Bereitstellung des ersten Kontaktmediums, die Kombination einer Ultraschallsonde mit einem auswechselbaren Aufsatz interessant, bei der beim Aufsetzen des Aufsatzes auf die Ultraschallsonde eine Membran des Aufsatzes so verformt wird, dass eine Vorspannung aufgebaut wird und dass der resultierende Ausgangsdruck auf die Ultraschallsonde mittels eines in der Ultraschallsonde integrierten Drucksensors (Kraftsensors) gemessen wird.

Ein Vergleich des gemessenen Ausgangsdrucks mit dem Soll-Ausgangsdruck kann nun dazu eingesetzt werden, den Drucksensor zu kalibrieren. Dies erfolgt mit einer entsprechenden I<alibrierungseinrichtung. Es kann sinnvoll sein, für den Kalibrierungsvorgang eine Orientierung der Ultraschallsonde (z. B. senkrecht nach unten) vorzugeben, um gravitative Einflüsse zu berücksichtigen. Grundsätzlich ist es möglich, die Ausrichtung zu messen (insbesondere mittels eines Lagesensors) und entweder die Kalibrierung nur dann durchzuführen, wenn die Ausrichtung korrekt ist oder bei der Kalibrierung die bestimmte Orientierung rechnerisch zu berücksichtigen.

Die I<alibrierungseinrichtung kann in der Ultraschallsonde oder in einem externen, mit der Sonde verbundenen Gerät angeordnet sein. Sie kann durch Softwaremodule und/oder dedizierte Hardware realisiert werden.

Die Messung des Ausgangsdrucks bzw. der Vergleich mit einem Soll-Ausgangsdruck kann zusätzlich oder alternativ auch dazu eingesetzt werden, das korrekte Aufsetzen des Aufsatzes zu erkennen, insbesondere anhand eines gemessenen Ausgangsdrucks in einem vorgegebenen Bereich. Die Ultraschallsonde kann so gesteuert werden, dass sie bzw. ein Messvorgang gestartet wird, sobald ein korrekt aufgesetzter Aufsatz erkannt wurde.

Die entsprechende Detektionsvorrichtung kann in der Ultraschallsonde oder in einem externen, mit der Sonde verbundenen Gerät angeordnet sein. Sie kann durch Softwaremodule und/oder dedizierte Hardware realisiert werden. Auch sie ist grundsätzlich unabhängig von der erfindungsgemässen Bereitstellung des ersten Kontaktmediums einsetzbar.

Eine Kalibrierung des Drucksensors kann - unabhängig von der erfindungsgemässen Bereitstellung des ersten Kontaktmediums - auch dadurch erfolgen, dass eine erste Druckmessung bei einer ersten räumlichen Orientierung des Ultraschalll<opfs erfolgt, während eine zweite Druckmessung bei einer zweiten räumlichen Orientierung des Ultraschalll<opfs erfolgt, wobei sich ein Winkel einer Längsachse des Ultraschalll<opfs zur Lotrechten zwischen der ersten räumlichen Orientierung und der zweiten räumlichen Orientierung um mindestens 45° unterscheidet, insbesondere weist in der ersten räumlichen Orientierung der Ultraschallkopf im Wesentlichen senkrecht nach unten und in der zweiten räumlichen Orientierung im Wesentlichen senkrecht nach oben, d. h. der Winl<elunterschied beträgt ca. 180°. Die bezüglich des Ultraschalll<opfs unterschiedlich gerichtete Gewichtskraft führt zu einem Unterschied zwischen dem bei der ersten Druckmessung ermittelten ersten Druck und dem bei der zweiten Druckmessung ermittelten zweiten Druck. Dieser Unterschied kann zur Kalibrierung des Drucksensors eingesetzt werden, er kann aber alternativ oder zusätzlich auch dazu dienen, das korrekte Aufsetzen des Aufsatzes zu erkennen.

Der Ultraschallkopf kann mit einem Lagesensor versehen sein, so dass die effektive Orientierung des Kopfs bei den Druckmessungen präzise bestimmt und bei der Kalibrierung bzw. der Detektion des korrekten Aufsetzens berücksichtigt werden kann. In diesem Fall können die beiden Orientierungen an sich beliebig sein, sofern sie sich in Bezug auf die einwirkende Gewichtskraft in Längsrichtung ausreichend unterscheiden. Wird die Orientierung für die beiden Messungen vorgegeben, ist ein Lagesensor nicht zwingend. So sind insbesondere die einwirkenden Kräfte im Bereich einer vertikalen Orientierung des Ultraschalll<opfs (nach unten bzw. nach oben) nur wenig sensitiv auf kleinere Winkelfehler, d. h. Abweichungen von 0° bzw. 180°.

Der Lagesensor kann ausser für die Kalibrierung grundsätzlich auch bei den effektiven Druckmessungen zum Einsatz kommen, um unterschiedliche Einflüsse der Schwerkraft bei unterschiedlichen Orientierungen zu berücksichtigen und die ermittelten Druckwerte entsprechend zu korrigieren.

Die I<alibrierungseinrichtung kann in der Ultraschallsonde oder in einem externen, mit der Sonde verbundenen Gerät angeordnet sein. Sie kann durch Softwaremodule und/oder dedizierte Hardware realisiert werden.

Bei einer bevorzugten Ausführungsform umfasst die Ultraschallsonde eine Leseeinrichtung zum Auslesen eines am aufgesetzten Aufsatz angeordneten Datenspeichers und eine Sperreinrichtung, die eine Nutzung der Ultraschallsonde mit dem Aufsatz nach Ablauf einer vorbestimmten Zeitspanne verhindert.

Beim Datenspeicher kann es sich insbesondere um einen passiven RFID-Transponder handeln, der berührungslos auslesbar ist. Es sind aber auch Speicher- oder Prozessorchips einsetzbar, die drahtlos kommunizieren oder über Kontakte am Aufsatz mit Gegenkontakten an der Ultraschallsonde zusammenwirken.

Die vorbestimmte Zeitspanne kann an einem fixen Ablaufdatum enden, oder es wird beim erstmaligen Aufsetzen des Aufsatzes ein Timer aktiviert, der nach der vorbestimmten Zeitspanne abläuft.

Die Sperreinrichtung kann in der Ultraschallsonde oder in einem externen, mit der Sonde verbundenen Gerät angeordnet sein. Sie kann durch Softwaremodule und/oder dedizierte Hardware realisiert werden.

Aus der nachfolgenden Detailbeschreibung und der Gesamtheit der Patentansprüche ergeben sich weitere vorteilhafte Ausführungsformen und Merkmalskombinationen der Erfindung.

### Kurze Beschreibung der Zeichnungen

Die zur Erläuterung des Ausführungsbeispiels verwendeten Zeichnungen zeigen:
- Fig. 1: eine Ausführungsform eines erfindungsgemässen Aufsatzes für eine Ultraschallsonde;
- Fig. 2: einen Querschnitt durch den Aufsatz;
- Fig. 3: die Ultraschallsonde mit aufgesetztem Aufsatz;
- Fig. 4A-D: Schrägansichten von Elementen einer Verpackung für den Aufsatz; und
- Fig. 5A-D: Querschnitte durch die Verpackung und den Aufsatz.

Grundsätzlich sind in den Figuren gleiche Teile mit gleichen Bezugszeichen versehen.

### Wege zur Ausführung der Erfindung

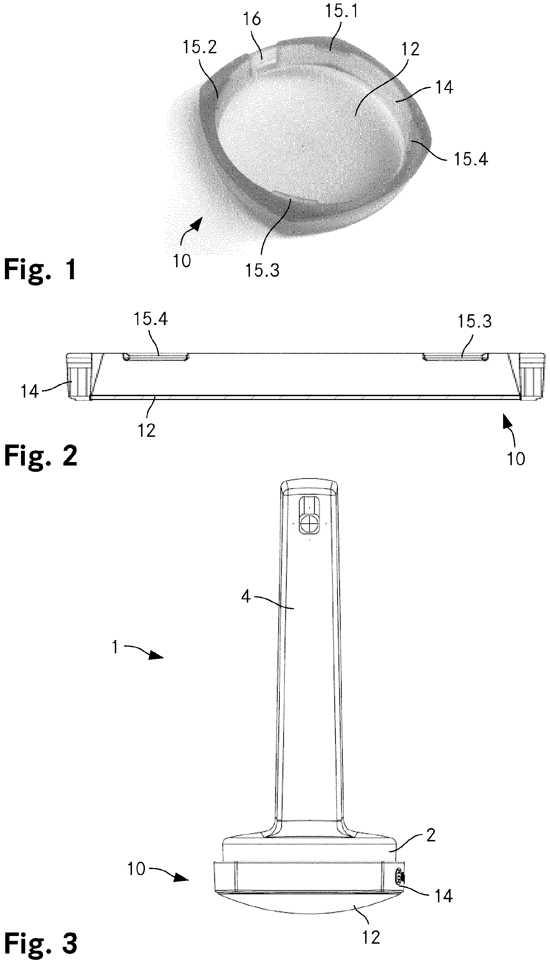

Die Figur 1 zeigt eine Ausführungsform eines erfindungsgemässen Aufsatzes für eine Ultraschallsonde, die Figur 2 zeigt einen Querschnitt durch den Aufsatz. Der Aufsatz 10 ist einstückig aus Silikon mit einer Härte von 40 Shore A gefertigt. Er umfasst eine im Wesentlichen kreisförmige Membran 12, an die rundum ein Mantelabschnitt 14 anschliesst. Der Mantelabschnitt setzt sich aus vier gleich langen gebogenen Segmenten zusammen, wobei der Krümmungsradius der Segmente jeweils etwas grösser ist als der Radius der Membran 12. Entsprechend ergeben sich an den Übergängen zwischen benachbarten Segmenten abgerundete Ecken. Die Dicke der Membran 12 beträgt konstant ca. 0.2 mm, diejenige des Mantelabschnitts 14 ca. 0.8 mm. An der der Membran 12 entgegengesetzten Seite des Mantelabschnitts 14 sind in den Eckbereichen vier Vorsprünge 15.1, 15.2, 15.3, 15.4 ausgebildet. Sie erstrecken sich ausgehend vom Mantelabschnitt 14 radial nach innen. Zwischen zwei der Vorsprünge 15.1, 15.2 ist auf der Innenseite des Mantelabschnitts 14 eine rechteckige Ausnehmung 16 ausgespart, die bis zu dem der Membran 12 entgegengesetzten Ende des Mantelabschnitts 14 reicht.

Die Figur 3 ist eine Seitenansicht der Ultraschallsonde 1 mit aufgesetztem Aufsatz 10. Die Ultraschallsonde 1 umfasst den Ultraschallkopf 2 und einen Griffteil 4. Im Ultraschallkopf ist der Piezoarray aufgenommen. Beim dargestellten Beispiel ist vor dem Piezoarray eine von einer flexiblen Kalotte vorne abgeschlossene Kammer mit einem ultraschalltransparenten Hydraulikmedium angeordnet. Die Geometrie der Kalotte entspricht insbesondere derjenigen eines Katenoids (also einem Rotationskörper einer I<ettenlinie). Die Kalotte ist aus einem Material mit einer Härte von 40 Shore A gefertigt, ihre Elastizität entspricht somit derjenigen der Membran 12 des Aufsatzes 10. Ein Druckmessfühler mündet in die Kammer, so dass ein von aussen über die Kalotte auf die Kammer ausgeübter Druck gemessen werden kann. Der Griffteil 4 nimmt die weiteren elektronischen Komponenten der Ultraschallsonde 1 auf.

Der Aufsatz 10 ist im Bereich des Ultraschalll<opfs 2 an der Ultraschallsonde 1 befestigt. Dazu bildet der Mantelabschnitt 14 einen Befestigungsbereich, wobei die Vorsprünge 15.1...4 mit entsprechenden Gegenstücken am Ultraschallkopf 2 zusammenwirken, um den Aufsatz 10 axial an der Ultraschallsonde 1 zu halten. Die Innenform des Mantelabschnitts 14 selbst ist der Aussenform des Ultraschalll<opfs 2 angepasst, so dass der Aufsatz 10 in radialer Richtung passend und der Ultraschallsonde 1 gehalten ist. Der Ultraschallkopf 2 weist zudem einen Vorsprung auf, der mit der Ausnehmung 16 im Aufsatz 10 bei korrekter Orientierung des Aufsatzes 10 zusammenwirkt und eine Anbringung des Aufsatzes 10 in anderen Orientierungen verhindert oder zumindest stark erschwert. Der Vorsprung des Kopfs und die Ausnehmung 16 des Aufsatzes 10 bilden somit eine Verdrehsicherung für den Aufsatz 10. Bei befestigtem Aufsatz 10 schmiegt sich die Membran 12 eng an die durch die Kalotte gebildete Stirnseite des Ultraschalll<opfs 2 an und nimmt insbesondere auch deren konvexe Form an. Der Raum zwischen der Kalotte und der Membran 12 wird umlaufend, durch das Zusammenwirken des Mantelabschnitts 14 und der Mantelfläche des Ultraschalll<opfs 2 abgedichtet.

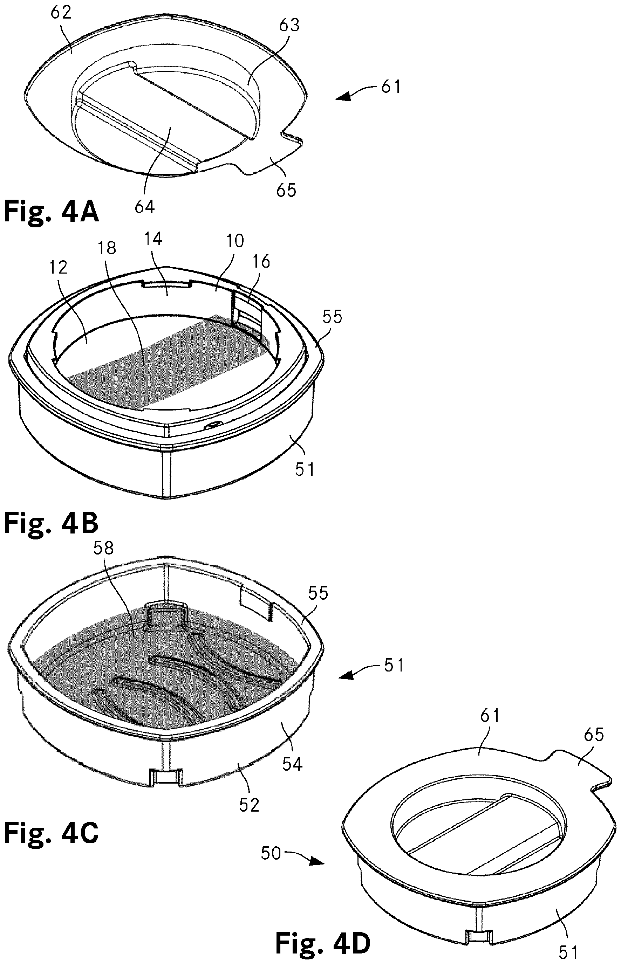

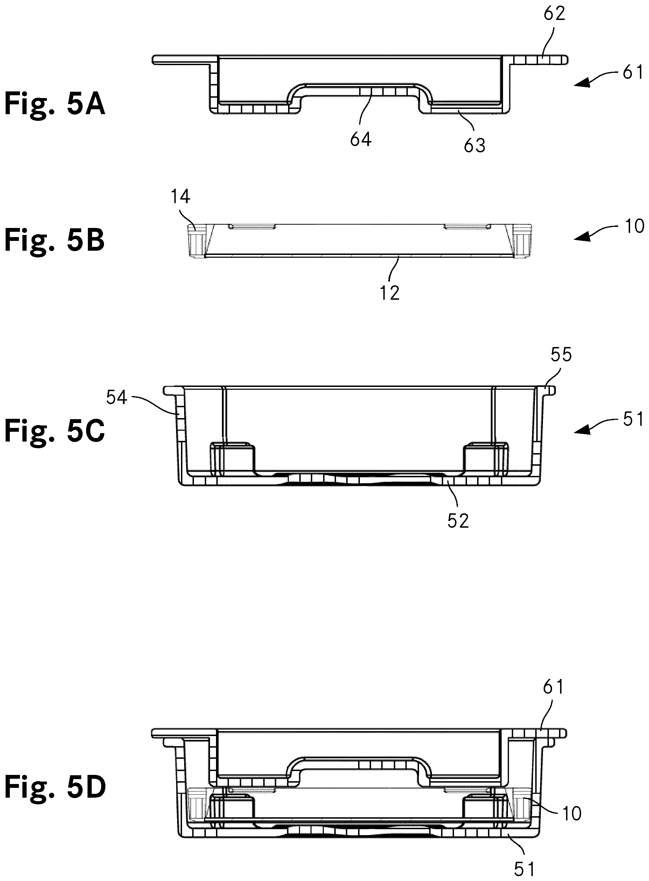

Die Figuren 4A-D sind Schrägansichten von Elementen einer Verpackung für den Aufsatz, und die Figuren 5A-D zeigen Querschnitte durch die Verpackung und den Aufsatz. Die Verpackung 50 umfasst einen Behälter 51 und einen Deckel 61. Der Behälter 51 ist becherartig ausgebildet und umfasst einen Boden 52 sowie einen davon aufragenden Mantel 54. Der Mantel 54 ist auf der dem Boden 52 entgegengesetzten Seite mit einem flanschartig nach aussen ragenden Rand 55 abgeschlossen. Die Form des Behälters 51 entspricht der Aussenform des Mantelabschnitts des Aufsatzes 10. Im Behälter 51 ist ein Gelbad 58 aufgenommen. Dieses wird durch eine Menge an sterilem Ultraschall-I<ontal<tgel gebildet, die den Boden 52 komplett bedeckt.

Wie in der Figur 4B gezeigt, kann der Aufsatz 10 passend im Behälter 51 aufgenommen werden. Die Aussenseite der Membran 12 wird dabei vom Gelbad 58 komplett benetzt. Auf der Innenseite der Membran 12 ist ein streifenförmiger Auftrag 18 mit sterilem Ultraschall-I<ontal<tgel angeordnet. Der Auftrag 18 erstreckt sich über den gesamten Durchmesser der Membran 12 und ist auf die Ausnehmung 16 ausgerichtet. Seine Breite entspricht ungefähr einem Drittel des Durchmessers. Im Bereich des Auftrags 18 ist die Membran vollflächig mit dem I<ontal<tgel bedeckt, wobei die Dicke der Gelschicht ca. 1 mm beträgt. Ausserhalb des Auftrags 18 ist keine Gelschicht vorhanden.

Mit dem Deckel 61 lässt sich der Behälter 51 mit aufgenommenem Aufsatz 10 verschliessen. Der Deckel 61 umfasst einen Randbereich 62, der sich beim Verschliessen auf dem Rand 55 des Behälters 51 und der oberen Seite des Aufsatzes 10 abstützt. Zentral ist auf der Innenseite des Deckels 61 ein kreisförmiger Absatz 63 angeordnet. Seine Aussenkontur entspricht der Innenkontur des Mantelabschnitts 14 des Aufsatzes 10. Im Absatz 63 ist wiederum eine längliche Ausnehmung 64 ausgebildet, die gegenüber der Stirnfläche des Absatzes 63 zurücktritt. Ihre Ausdehnung entspricht im Wesentlichen der Ausdehnung des streifenförmigen Auftrags 18 auf der Innenseite der Membran 12. Der Bereich des Absatzes 63 ausserhalb der Ausnehmung 64 kontaktiert im verpackten Zustand die Innenseite der Membran 12 vollflächig und verhindert so, dass sich das Ultraschall-I<ontal<tgel des Auftrags 18 in weitere Bereiche der Membran 12 verteilt. Der Deckel 61 umfasst schliesslich eine an den Rand 62 anschliessende Lasche 65, mit der sich der Deckel 61 manuell einfach und schnell entfernen lässt.

Damit mit der in der Ultraschallsonde integrierten Druckmesseinrichtung präzise Druckmessungen erfolgen können, sollte diese regelmässig kalibriert werden. Bei einem ersten I<alibrierverfahren erfolgt eine solche Kalibrierung anhand eines Ausgangsdrucks, der durch die Vorspannung der Membran des erfindungsgemässen Aufsatzes bewirkt wird. Im Kalibriermodus wird der Anwender angewiesen, den Aufsatz auf die Ultraschallsonde aufzusetzen und diese in einer bestimmten Orientierung (z. B. mit der Stirnseite vertikal nach unten) zu halten, wobei die Membran frei, ohne äussere Krafteinwirkung, bleiben soll. Die Aufsätze sind so gefertigt, dass sich beim Zusammenwirken mit der Ultraschallsonde ein bekannter Druckwert ergibt (z. B. im Bereich 3-10 mmHg). Weicht der in dieser Konfiguration von der Druckmesseinrichtung bestimmte Wert von diesem bekannten Druckwert ab, kann aus der entsprechenden Differenz ein Korrekturwert bestimmt werden. Im einfachsten Fall handelt es sich um einen Offset-Wert, mit dem künftige Druckmessungen, bis zu einer erneuten Kalibrierung, korrigiert werden.

Es ist möglich, eine sondenspezifische Erstkalibrierung durchzuführen, insbesondere herstellerseitig, wobei der von den Aufsätzen erzeugte Druckwert für die spezifische Sonde präzise bestimmt und in der Sonde, einem Steuergerät und/oder einem Server hinterlegt wird.

Ebenfalls ist es möglich, im Kalibriermodus auch bereits vor dem Aufsetzen des Aufsatzes eine erste Druckmessung durchzuführen. Aus den unterschiedlichen Druckwerten vor und nach dem Aufsetzen lassen sich weitere Korrekturparameter bestimmen, z. B. ein Korrekturfaktor, der zusammen mit dem Offset zur Korrektur der Druckmessungen herangezogen wird.

Der Druckwert nach dem Aufsetzen (oder die Differenz zum Druckwert vor dem Aufsetzen) kann auch zur Erkennung genutzt werden, ob der Aufsatz korrekt auf die Ultraschallsonde aufgesetzt wurde. Bei einem zu geringen oder zu hohen Druckwert oder einer zu geringen bzw. zu hohen Differenz wird eine Warnung ausgegeben und der Anwender angewiesen, die korrekte Befestigung des Aufsatzes zu prüfen.

Eine weitere mögliche Art der Kalibrierung ist grundsätzlich unabhängig vom erfindungsgemässen Aufsatz, kann aber ohne weiteres auch bei Verwendung des Aufsatzes genutzt werden. Es ist auch möglich, diesen weiteren Ansatz in Kombination mit den vorgenannten I<alibrierungsschritten einzusetzen. Dabei werden mindestens zwei Druckwertebei unterschiedlichen Orientierungen der Ultraschallsonde bestimmt. Aus diesen Druckwerten können dann Korrekturwerte bestimmt werden.

Beispielsweise wird im Kalibriermodus der Benutzer angewiesen, die Sonde zunächst mit der Stirnseite senkrecht nach oben zu richten, wonach eine erste Messung durchgeführt wird. Anschliessend erfolgt eine zweite Messung mit nach unten gerichteter Stirnseite. Weil die Wirkung der auf die für die Druckmessung relevanten Massen wirkenden Gewichtskraft entgegengesetzt ist, werden sich unterschiedliche Druckwerte ergeben. Da die Massen (Elemente der Ultraschallsonde und gegebenenfalls Elemente des Aufsatzes) bekannt sind, lässt sich die Druckmesseinrichtung anhand der gemessenen Drucke und/oder anhand einer Druckdifferenz kalibrieren.

Die Erfindung ist nicht auf das dargestellte Ausführungsbeispiel beschränkt. So kann insbesondere auch ein dünneres Membranmaterial zum Einsatz kommen. Der ringartige Befestigungsbereich kann durch ein Element aus einem anderen Material gebildet sein, in dem die Membran befestigt, z. B. eingespannt, ist. Seine Geometrie kann an verschiedene Ultraschallköpfe mit linearen oder gekrümmten Arrays angepasst sein, seine Form kann somit auch derjenigen einer Ellipse, eines Rechtecks o. ä. entsprechen. Die Befestigung des Aufsatzes am Ultraschallkopf kann zudem auf andere Weise erfolgen, und der Aufsatz kann mantelseitige Abschnitte oder Komponenten umfassen, die sich bei befestigtem Aufsatz weiter nach hinten, entlang des Handgriffs der Ultraschallsonde, erstrecken. So kann die Übertragung von Keimen noch effektiver verhindert werden.

Auch die Verpackung kann anders ausgeführt werden. Sie kann insbesondere aus unterschiedlichsten Materialien, z. B. Karton, (Bio-)Kunststoffen, geschäumten Materialien usw., bzw. Materialkombinationen, gefertigt werden.

Zusammenfassend ist festzustellen, dass die Erfindung einen Aufsatz schafft, der einfach zu handhaben ist und eine hohe Signalqualität ermöglicht.

## Patentansprüche

1. Auswechselbarer Aufsatz (10) für eine Ultraschallsonde (1), umfassend
a) einen ringartigen Befestigungsbereich (14) zur Befestigung des Aufsatzes (10) an der Ultraschallsonde (1);
b) eine am Befestigungsbereich (14) angeordnete flexible Membran (12);
wobei ein erstes gelartiges Kontaktmedium auf einer innenseitigen I<ontaktfläche der Membran (12) aufgebracht ist, **dadurch gekennzeichnet dass** die Kontaktfläche bei an der Ultraschallsonde (1) befestigtem Aufsatz (10) eine Stirnseite der Ultraschallsonde (1) kontaktiert;
dass vor dem Befestigen des Aufsatzes (10) das erste Kontaktmedium auf der Kontaktfläche derart verteilt ist, dass in einem Arraybereich der Kontaktfläche eine Menge des ersten Kontaktmediums pro Fläche mindestens doppelt so gross ist wie in einem übrigen Bereich der Kontaktfläche, wobei eine Positionierung und Ausdehnung des Arraybereichs einer Positionierung und Ausdehnung eines Piezoarrays der Ultraschallsonde (1) angepasst ist und eine Fläche des Arraybereichs mindestens 60% und maximal 175% einer Fläche des Piezoarrays beträgt.

2. Aufsatz (10) nach Anspruch 1, **dadurch gekennzeichnet, dass** der Arraybereich im Wesentlichen ganzflächig vom ersten Kontaktmedium bedeckt ist und dass der übrige Bereich der I<ontaktfläche im Wesentlichen frei von Kontaktmedium ist.

3. Aufsatz (10) nach Anspruch 1 oder 2, **dadurch gekennzeichnet, dass** der Arraybereich im Wesentlichen rechteckig ist.

4. Aufsatz (10) nach einem der Ansprüche 1 bis 3, **dadurch gekennzeichnet, dass** auf einer Aussenfläche der Membran (12) vor dem Befestigen des Aufsatzes (10) ein zweites Kontaktmedium aufgebracht ist.

5. Aufsatz (10) nach einem der Ansprüche 1 bis 4, **gekennzeichnet durch** ein Garantieelement mit einer Geometrie, die dazu angepasst ist, bei einem Abziehen des an der Ultraschallsonde (1) befestigten Aufsatzes (10) mit einer Aufsetzgeometrie der Ultraschallsonde (1) so zusammenzuwirken, dass das Garantieelement mechanisch beschädigt wird.

6. Verpackter Aufsatz (10), umfassend einen Aufsatz (10) nach einem der Ansprüche 1 bis 5 und eine Verpackung (50), **dadurch gekennzeichnet, dass** die Verpackung (50) einen becherartigen Grundteil (51) und einen Deckel (61) umfasst, wobei eine Innengeometrie des becherartigen Grundteils (51) einer Aussengeometrie des Aufsatzes (10) angepasst ist, so dass dieser passend im Grundteil (51) aufnehmbar ist und dass der Deckel (61) eine Aussengeometrie aufweist, die einer Innengeometrie des Aufsatzes (10) angepasst ist, so dass der Deckel (61) bei im Grundteil (51) aufgenommenem Aufsatz (10) passend in den Aufsatz (10) einsetzbar ist.

7. Verpackter Aufsatz (10) nach Anspruch 6, **dadurch gekennzeichnet, dass** der Deckel (61) oder der Grundteil (51) auf einer Innenseite, die bei in der Verpackung (50) aufgenommenem Aufsatz (10) parallel zur Membran (12) des Aufsatzes (10) verläuft, eine Ausnehmung (64) aufweist, deren Geometrie einer Geometrie des Arraybereichs angepasst ist.

8. Verpackter Aufsatz (10) nach Anspruch 7, **dadurch gekennzeichnet, dass** bei zwischen dem Grundteil (51) und dem Deckel (61) angeordneten Aufsatz (10) die I<ontaktfläche des Aufsatzes (10) ausserhalb eines durch die Ausnehmung (64) definierten Bereichs derart vollflächig kontaktiert wird, dass ein Auslaufen des ersten Kontaktmediums aus dem Arraybereich in den übrigen Bereich gehemmt wird.

9. Verpackter Aufsatz (10) nach einem der Ansprüche 6 bis 8, **dadurch gekennzeichnet, dass** im Grundteil (51) eine Menge des zweiten Kontaktmediums enthalten ist, so dass die Aussenfläche der Membran (12) des aufgenommenen Aufsatzes (10) grossflächig im zweiten Kontaktmedium eingetaucht ist.

10. Ultraschallsonde (1) mit einem Aufsatz (10) nach einem der Ansprüche 1 bis 5.

11. Ultraschallsonde (1) nach Anspruch 10, **dadurch gekennzeichnet, dass** der Befestigungsbereich (14) des Aufsatzes (10) eine Kontur zum Zusammenwirken mit einer Gegenkontur der Ultraschallsonde (1) aufweist, wobei die Kontur und die Gegenkontur so ausgebildet sind, dass der Aufsatz (10) nur in einer oder zwei einander entgegengesetzten Orientierungen auf die Ultraschallsonde (1) aufsetzbar ist.

12. Ultraschallsonde (1) nach Anspruch 10 oder 11, **dadurch gekennzeichnet, dass** die Stirnseite eine konvexe Geometrie aufweist und dass die Membran (12) des Aufsatzes (10) beim Aufsetzen gemäss der konvexen Geometrie verformt wird.

13. Ultraschallsonde (1) nach Anspruch 12, **dadurch gekennzeichnet, dass** durch die Verformung eine Vorspannung in der Membran (12) aufgebaut wird und dass die Ultraschallsonde (1) einen Drucksensor zum Messen eines über den aufgesetzten Aufsatz (10) aufgrund der Vorspannung auf die Ultraschallsonde (1) ausgeübten Ausgangsdrucks umfasst.

14. Ultraschallsonde (1) nach Anspruch 13, **gekennzeichnet durch** eine I<alibrierungseinrichtung, die dazu angepasst ist, den Drucksensor anhand des Ausgangsdrucks zu kalibrieren.

15. Ultraschallsonde (1) nach Anspruch 13 oder 14, **gekennzeichnet durch** eine Detektionsvorrichtung, die dazu angepasst ist, ein korrektes Aufsetzen des Aufsatzes (10) anhand des gemessenen Ausgangsdrucks zu erkennen.

16. Ultraschallsonde (1) nach einem der Ansprüche 10 bis 15, **gekennzeichnet durch** eine Leseeinrichtung zum Auslesen eines am aufgesetzten Aufsatz (10) angeordneten Datenspeichers und durch eine Sperreinrichtung, die eine Nutzung der Ultraschallsonde (1) mit dem Aufsatz (10) nach Ablauf einer vorbestimmten Zeitspanne verhindert.

## Claims

1. Replaceable attachment (10) for an ultrasonic probe (1), comprising
a) an annular mounting region (14) for mounting the attachment (10) on the ultrasonic probe (1);
b) a flexible membrane (12) arranged on the mounting region (14);
wherein a first gel-like contact medium is applied to an inside facing contact surface of the membrane (12),
**characterized in that**,
when the attachment (10) is attached to the ultrasonic probe (1), the contact surface contacts an end face of the ultrasonic probe (1);
before the attachment (10) is attached, the first contact medium is distributed on the contact surface in such a way that, in an array region of the contact surface, an amount of the first contact medium per unit area is at least twice as large as in a remaining region of the contact surface, wherein a positioning and extent of the array region is adapted to the positioning and extent of a piezoelectric array of the ultrasonic probe (1), and an area of the array region is at least 60% and at most 175% of an area of the piezoelectric array.

2. Attachment (10) according to claim 1, **characterized in that** the array region is covered essentially over its entire surface area by the first contact medium and **in that** the remaining region of the contact surface is essentially free of contact medium.

3. Attachment (10) according to claim 1 or 2, **characterized in that** the array region is essentially rectangular.

4. Attachment (10) according to one of claims 1 to 3, **characterized in that** a second contact medium is applied to an outer surface of the membrane (12) before the attachment (10) is mounted.

5. Attachment (10) according to one of claims 1 to 4, **characterized by** a guarantee element with a geometry that is adapted to interact with an attachment geometry of the ultrasonic probe (1) when the attachment (10) attached to the ultrasonic probe (1) is pulled off, in such a way that the guarantee element is mechanically damaged.

6. Packaged attachment (10), comprising an attachment (10) according to one of Claims 1 to 5 and a package (50), **characterized in that** the package (50) comprises a cup-like base part (51) and a lid (61), wherein an inner geometry of the cup-like base part (51) is adapted to an outer geometry of the attachment (10) so that it can be fittingly accommodated in the base part (51) and that the lid (61) has an outer geometry that is adapted to an inner geometry of the attachment (10) so that the lid (61) can be fittingly inserted into the attachment (10) when the attachment (10) is accommodated in the base part (51).

7. Packaged attachment (10) according to claim 6, **characterized in that** the lid (61) or the base part (51) has, on an inner side which extends parallel to the membrane (12) of the attachment (10) when the attachment (10) is held in the package (50), a recess (64) whose geometry is adapted to a geometry of the array region.

8. Packaged attachment (10) according to claim 7, **characterized in that**, with the attachment (10) arranged between the base part (51) and the lid (61), the contact surface of the attachment (10) is contacted over its entire surface outside of a region defined by the recess (64) in such a way that leakage of the first contact medium from the array region into the remaining region is inhibited.

9. Packaged attachment (10) according to one of claims 6 to 8, **characterized in that** an amount of the second contact medium is contained in the base part (51) so that the outer surface of the membrane (12) of the received attachment (10) is immersed in the second contact medium over a large area.

10. Ultrasonic probe (1) with an attachment (10) according to one of claims 1 to 5.

11. Ultrasonic probe (1) according to claim 10, **characterized in that** the mounting region (14) of the attachment (10) has a contour for interacting with a countercontour of the ultrasonic probe (1), wherein the contour and the countercontour are configured such that the attachment (10) can be placed on the ultrasonic probe (1) only in one or two opposing orientations.

12. Ultrasonic probe (1) according to claim 10 or 11, **characterized in that** the end face has a convex geometry and **in that** the membrane (12) of the attachment (10) is deformed in accordance with the convex geometry when it is attached.

13. Ultrasonic probe (1) according to claim 12, **characterized in that** a prestress is built up in the membrane (12) by the deformation and **in that** the ultrasonic probe (1) comprises a pressure sensor for measuring an output pressure exerted on the ultrasonic probe (1) via the attached attachment (10) due to the prestress.

14. Ultrasonic probe (1) according to claim 13, **characterized by** a calibration device adapted to calibrate the pressure sensor on the basis of the output pressure.

15. The ultrasonic probe (1) according to claims 13 or 14, **characterized by** a detection device that is adapted to detect correct placement of the attachment (10) based on the measured output pressure.

16. Ultrasonic probe (1) according to one of claims 10 to 15, **characterized by** a reading device for reading a data storage arranged on the attached attachment (10) and by a blocking device which prevents a usage of the ultrasonic probe (1) with the attachment (10) after a predetermined period of time has elapsed.

## Revendications

1. Embout (10) interchangeable, destiné à une sonde à ultrasons (1), comprenant
a) une zone de fixation (14) de forme annulaire pour la fixation de l'embout (10) sur la sonde à ultrasons (1) ;
b) une membrane (12) souple, placée sur la zone de fixation (14) ;
un premier agent de contact de type gel étant appliqué sur une surface de contact interne de la membrane (12), **caractérisé en ce que** lorsque l'embout (10) est fixé sur la sonde à ultrasons (1), la surface de contact est en contact avec une face frontale de la sonde à ultrasons (1) ;
**en ce qu'**avant la fixation de l'embout (10), le premier agent de contact est distribué sur la surface de contact de telle sorte, que dans une zone de matrice de la surface de contact, une quantité du premier agent de contact par surface corresponde au moins au double de celle dans la zone restante de la surface de contact, un positionnement et une étendue de la zone de matrice étant adaptés à un positionnement et à une étendue d'une matrice piézoélectrique de la sonde à ultrasons (1) et qu'une surface de la zone de matrice s'élève à au moins 60 % et au maximum à 175 % d'une surface de la matrice piézoélectrique.

2. Embout (10) selon la revendication 1, **caractérisé en ce que** la zone de matrice est recouverte sensiblement à pleine surface par le premier agent de contact et **en ce que** la zone restante de la surface de contact est sensiblement exempte d'agent de contact.

3. Embout (10) selon la revendication 1 ou 2, **caractérisé en ce que** la zone de matrice est sensiblement rectangulaire.

4. Embout (10) selon l'une quelconque des revendications 1 à 3, **caractérisé en ce que** sur une surface externe de la membrane (12), un deuxième agent de contact est appliqué avant la fixation de l'embout (10).

5. Embout (10) selon l'une quelconque des revendications 1 à 4, **caractérisé par** un élément de garantie, présentant une géométrie qui est adaptée pour coopérer avec la sonde à ultrasons (1), lors d'un retrait de l'embout (10) fixé sur la sonde à ultrasons (1), de telle sorte que l'élément de garantie soit mécaniquement altéré.

6. Embout emballé (10), comprenant un embout (10) selon l'une quelconque des revendications 1 à 5 et un emballage (50), **caractérisé en ce que** l'emballage (50) comprend une partie de base (51) en forme de godet et un couvercle (61), une géométrie interne de la partie de base (51) en forme de godet étant adaptée à une géométrie externe de l'embout (10), de sorte que celui-ci puisse être réceptionné de manière adaptée dans la partie de base (51) et que le couvercle (61) présente une géométrie externe, qui est adapté à la géométrie interne de l'embout (10), de telle sorte que lorsque l'embout (10) est réceptionné dans la partie de base (51), le couvercle (61) soit insérable de manière adaptée dans l'embout (10).

7. Embout emballé (10) selon la revendication 6, **caractérisé en ce que** sur une face interne, qui lorsque l'embout (10) est réceptionné dans l'emballage (50) s'écoule à la parallèle de la membrane (12) de l'embout (10), le couvercle (61) ou la partie de base (51) comporte un évidement (64) dont la géométrie est adaptée à une géométrie de la zone de matrice.

8. Embout emballé (10) selon la revendication 7, caractérisé en ce lorsque l'embout (10) est placé entre la partie de base (51) et le couvercle (61), la surface de contact de l'embout (10) est mise en contact à pleine surface à l'extérieur d'une zone définie par l'évidement (64) de telle sorte qu'un écoulement du premier agent de contact à partir de la zone de matrice dans la zone restante soit inhibé.

9. Embout emballé (10) selon l'une quelconque des revendications 6 à 8, **caractérisé en ce que** dans la partie de base (51) est contenue une quantité du deuxième agent de contact, de telle sorte que la surface externe de la membrane (12) de l'embout (10) réceptionné soit immergée à grande surface dans le deuxième agent de contact.

10. Sonde à ultrasons (1), pourvue d'un embout (10) selon l'une quelconque des revendications 1 à 5.

11. Sonde à ultrasons (1) selon la revendication 10, **caractérisée en ce que** la zone de fixation (14) de l'embout (10) comporte un contour destiné à coopérer avec un contour antagoniste de la sonde à ultrasons (1), le contour et le contour antagoniste étant conçus de telle sorte que l'embout (10) ne soit emboîtable sur la sonde à ultrasons (1) que selon une ou deux orientations mutuellement opposées.

12. Sonde à ultrasons (1) selon la revendication 10 ou 11, **caractérisée en ce que** la face frontale présente une géométrie convexe et **en ce que** lors de l'emboîtement, la membrane (12) de l'embout (10) est déformée selon la géométrie convexe.

13. Sonde à ultrasons (1) selon la revendication 12, **caractérisée** en ce par la déformation, une précontrainte s'établit dans la membrane (12) et en ce que la sonde à ultrasons (1) comprend un capteur de pression destiné à mesurer une pression de sortie exercée sur la sonde à ultrasons (1) par l'intermédiaire de l'embout (10) emboîté, du fait de la précontrainte.

14. Sonde à ultrasons (1) selon la revendication 13, **caractérisée par** un système de calibrage, qui est adapté pour calibrer le capteur de pression à l'aide de la pression de sortie.

15. Sonde à ultrasons (1) selon la revendication 13 ou 14, **caractérisée par** un dispositif de détection, qui est adapté pour identifier un emboîtement correct de l'embout (10) à l'aide de la pression de sortie mesurée.

16. Sonde à ultrasons (1) selon l'une quelconque des revendications 10 à 15, **caractérisée par** un système de lecture, destiné à lire une mémoire de données placée sur l'embout (10) emboîté et par un système de blocage, qui empêche une utilisation de la sonde à ultrasons (1) avec l'embout (10), après l'écoulement d'une certaine période.
